# EUROPEAN PATENT APPLICATION

(11) **EP 4 501 236 A1**
(43) Date of publication of application: **05.02.2025**
(21) Application number: 23778417.8
(22) Date of filing: 31.03.2023
(51) Int. Cl.: A61B 5/294, A61B 5/291, A61B 5/293, A61B 5/388

(54) **IMPLANTABLE STRETCHABLE FLEXIBLE NEURAL ELECTRODE, ELECTRODE GROUP, AND ELECTRODE ARRAY**

(30) Priority: 02.04.2022 CN 202210351191
(71) Applicant: Beijing BCIFlex Medical Technology Co., Ltd., Beijing 100190 (CN)
(72) Inventor: FANG, Ying, Beijing 100190 (CN); TIAN, Huihui, Beijing 100190 (CN); FANG, Runjiu, Beijing 100190 (CN); DU, Yan, Beijing 100190 (CN)
(74) Representative: Gulde & Partner
(86) International application number: PCT/CN2023/085295
(87) International publication number: WO 2023/186052

(57) **Abstract**

An implantable stretchable flexible neural electrode, electrode group, and electrode array, manufacturing methods, and implantation methods. The implantable stretchable flexible neural electrode comprises an electrode wire (10) and a flexible insulating part (20) covering the electrode wire (10). The flexible neural electrode further comprises: a spiral structure (SS) of the electrode wire (10) and the flexible insulating part (20); the spiral structure (SS) can be stretched; an electrode point (30) and an auxiliary implantation structure (40) are provided at the spiral structure (SS); the electrode point (30) is electrically connected to the electrode wire (10) and is exposed from the flexible insulating part (20); the auxiliary implantation structure (40) is configured to, under the action of an external force, drive the spiral structure (SS) to stretch. The achievable length of the flexible neural electrode in the effective area can be greatly improved, the flexibility of operation of implanting the flexible neural electrode into a target tissue is improved, and the stability of the flexible neural electrode after the flexible neural electrode is implanted into the target tissue is ensured.

## Description

This application claims the priority of Chinese Patent Application No.202210351191.0 filed on April 2, 2022, and the disclosure of the above-mentioned Chinese Patent Application is hereby incorporated in its entirety as a part of this application.

### TECHNICAL FIELD

At least one embodiment of the present disclosure relates to an implantable stretchable flexible neural electrode, an implantable stretchable flexible neural electrode group, an implantable stretchable flexible neural electrode array, as well as a manufacturing method and an implantation method thereof.

### BACKGROUND

The main function of neural electrode is to realize mutual conversion between bioelectrical signals with ions as carriers and universal electrical signals with electrons as carriers, so as to realize the recording and regulation of brain signals and peripheral nerve signals. The existing neural electrodes include electroencephalogram (EEG) electrodes, electrocorticography (ECoG) electrodes and implantable electrodes. Implantable neural electrodes can record and regulate the electrical activities of multiple neurons, and hence have a broad application prospect in the fields of nervous system disease treatment and brain-computer interface, etc.

At present, the most widely used implantable neural electrode is silicon-based rigid neural electrode. However, the mechanical properties of rigid neural electrodes do not match the brain, causing great mechanical damage. At the same time, due to the brain's own activities, the rigid electrode is easy to move slightly in the brain, which leads to the instability of neural signal recording and aggravates the inflammatory reaction of the brain. As a result, colloid cells are generated around the electrode and attached onto the surface of the electrode site, leading to the attenuation of electrical signals and the failure of the electrode site. Therefore, it is difficult for rigid electrodes to ensure the long-term stability of signal recording. Compared with the rigid neural electrode, the mechanical properties of the flexible neural electrode match the large target tissue, which greatly reduces the movement with the target tissue and the inflammatory reaction of the target tissue, thus realizing long-term stable recording and regulation of nerve signals.

At present, the structure of flexible neural electrode is mostly based on linear electrode wire structure, which limits the high-throughput transfer and in-situ implantation of electrode wire. Moreover, the implantation depth of flexible linear electrode wire is limited by the size of processing technology and the movable distance of the electrode.

### SUMMARY

According to an embodiment of the present disclosure, an implantable stretchable flexible neural electrode is provided, including an electrode wire and a flexible insulating part wrapping the electrode wire, wherein the flexible neural electrode includes a spiral structure of the electrode wire and the flexible insulating part, wherein the spiral structure is stretchable; an electrode site and an auxiliary implantation structure are provided at the spiral structure; the electrode site is electrically connected to the electrode wire and exposed from the flexible insulating part; and the auxiliary implantation structure is configured to drive the spiral structure to stretch under an action of external force.

For example, the spiral structure is configured to be stretchable in any direction.

For example, the spiral structure includes a plurality of circles, wherein any two adjacent circles among the plurality of circles are an inner circle and an outer circle, respectively, the outer circle surrounds the inner circle, and a termination end of the outer circle is connected to an initial end of the inner circle.

For example, the auxiliary implantation structure is provided at an innermost circle among the plurality of circles.

For example, the flexible insulating part is provided with a protruding part extending beyond the electrode wire, and the auxiliary implantation structure is a through hole, a groove or a protrusion provided at the protruding part.

For example, a position mark is provided on the protruding part.

For example, each of the plurality of circles includes a plurality of curved portions connected in sequence.

For example, the implantable stretchable flexible neural electrode according to the embodiment of the present disclosure further includes a linear structure of the electrode wire and the flexible insulating part, wherein the linear structure is connected to the spiral structure.

For example, the linear structure includes an auxiliary electrode wire wrapped by the flexible insulating part; and in the linear structure, the auxiliary electrode wire is electrically connected to the electrode wire.

For example, the implantable stretchable flexible neural electrode according to the embodiment of the present disclosure further includes an anchoring structure, wherein an angle is provided between the anchoring structure and the electrode wire, and the anchoring structure is configured to interact with a target tissue after the flexible neural electrode is implanted into the target tissue so as to prevent the flexible neural electrode from moving relative to the target tissue.

For example, an edge of the anchoring structure extending from the electrode wire in a direction away from the electrode wire is in an arc shape, and the arc shape protrudes towards the auxiliary implantation structure.

For example, the anchoring structure includes an anchoring electrode wire covered by the flexible insulating part, and the electrode site is electrically connected to the electrode wire through the anchoring electrode wire.

For example, a material of the electrode wire and a material of the electrode site include at least one of gold, platinum and iridium; and a material of the flexible insulating part includes at least one of polyimide, parylene and SU-8 photoresist.

For example, the implantable stretchable flexible neural electrode according to the embodiment of the present disclosure further includes an adhesive layer provided between the electrode wire and the flexible insulating part.

For example, the implantable stretchable flexible neural electrode according to an embodiment of the present disclosure includes a plurality of electrode wires wrapped by the flexible insulating part and insulated from each other; and a plurality of electrode sites exposed from the flexible insulating part, electrically connected to the plurality of the electrode wires in one-to-one correspondence, and spatially separated from each other.

For example, the plurality of electrode wires are located at a same layer; or the plurality of electrode wires are located at a plurality of layers arranged in a stacked manner, and each of the plurality of layers includes at least one of the plurality of electrode wires.

For example, any two adjacent layers among the plurality of layers are a first layer and a second layer; in a stacking direction of the plurality of layers, the electrode wire located at the first layer and the electrode wire located at the second layer at least partially overlap or at least partially do not overlap.

For example, the plurality of electrode sites are located at a same side or at different sides of the flexible neural electrode.

According to an embodiment of the present disclosure, an implantable stretchable flexible neural electrode group is provided, including a plurality of flexible neural electrodes as described above, wherein the plurality of flexible neural electrodes are insulated from each other; a plurality of spiral structures of the plurality of flexible neural electrodes forms a composite spiral structure; and in a same circle of the composite spiral structure, parts of the plurality of spiral structures located in the same circle of the composite spiral structure are sequentially arranged from inside to outside.

For example, a plurality of electrode sites of the plurality of flexible neural electrodes are spatially separated from each other.

For example, the composite spiral structure includes a plurality of circles, wherein any two adjacent circles among the plurality of circles are an inner circle and an outer circle, respectively, the outer circle surrounds the inner circle, and a termination end of the outer circle is connected to an initial end of the inner circle; in each of the plurality of circles of the composite spiral structure, corresponding parts of the plurality of spiral structures are sequentially arranged from inside to outside in a same order.

For example, a plurality of auxiliary implantation structures of the plurality of flexible neural electrodes are integrated into a common auxiliary structure, and the common auxiliary structure is configured to drive a plurality of spiral structures of the plurality of flexible neural electrodes to stretch synchronously under an action of external force.

For example, a plurality of flexible insulating parts of the plurality of flexible neural electrodes are at least partially connected to each other.

For example, a plurality of auxiliary implantation structures of the plurality of flexible neural electrodes are spatially separated from each other.

According to an embodiment of the present disclosure, an implantable stretchable flexible neural electrode array is provided, including a plurality of flexible neural electrodes as described above, wherein the plurality of flexible neural electrodes are insulated from each other, and a plurality of spiral structures of the plurality of flexible neural electrodes are arranged in an array.

For example, each of the plurality of flexible neural electrodes includes a linear structure of the electrode wire and the flexible insulating part, and the linear structure is connected to the spiral structure.

According to an embodiment of the present disclosure, an implantable stretchable flexible neural electrode array is provided, including a plurality of flexible neural electrode groups as described above, wherein a plurality of composite spiral structures of the plurality of flexible neural electrode groups are arranged in an array.

For example, each of all the flexible neural electrodes included in the plurality of flexible neural electrode groups includes a linear structure of the electrode wire and the flexible insulating part, and the linear structure is connected to the spiral structure.

According to an embodiment of the present disclosure, a manufacturing method of the implantable stretchable flexible neural electrode or the implantable stretchable flexible neural electrode group or the implantable stretchable flexible neural electrode array as described above is provided, including: providing a base substrate, wherein the base substrate includes a first region and a second region; forming a first insulating layer on the base substrate; forming a conductive layer on the first insulating layer and patterning the conductive layer to form the electrode wire and the electrode site in the first region of the base substrate; forming a second insulating layer on the electrode wire and the electrode site; patterning the first insulating layer and the second insulating layer to form the auxiliary implantation structure and the flexible insulating part wrapping the electrode wire, and patterning the second insulating layer to expose the electrode site; and removing at least a portion of the base substrate located in the first region.

For example, the method according to the embodiment of the present disclosure further includes: forming a sacrificial layer at least in the first region of the base substrate before forming the first insulating layer on the base substrate; and the removing at least a portion of the base substrate located in the first region includes: removing the sacrificial layer and cutting the base substrate to remove at least a portion of the base substrate located in the first region.

For example, patterning the conductive layer to form a pad in the second region of the base substrate while forming the electrode wire and the electrode site in the first region of the base substrate, wherein the electrode wire is electrically connected to the pad; and the method further includes: patterning the second insulating layer to expose the pad.

According to an embodiment of the present disclosure, an implantation method of the implantable stretchable flexible neural electrode or the implantable stretchable flexible neural electrode group or the implantable stretchable flexible neural electrode array as described above is provided, including: applying an external force to the auxiliary implantation structure by using an auxiliary implantation instrument to implant at least part of the flexible neural electrode into a target tissue, and at least partially stretching the spiral structure under a drive of the external force during a process of implanting the at least part of the flexible neural electrode into the target tissue; and removing the auxiliary implantation instrument and leaving, in the target issue, the at least part of the flexible neural electrode implanted in the target tissue.

### BRIEF DESCRIPTION OF DRAWINGS

In order to explain the technical solution of the embodiments of the present disclosure more clearly, the accompanying drawings of the embodiments will be briefly introduced below. Obviously, the accompanying drawings in the following description only relate to some embodiments of the present disclosure, and are not intended to limit the present disclosure.
Fig. 1 is a first plan view of an implantable stretchable flexible neural electrode according to an embodiment of the present disclosure;
Fig. 2 is a second plan view of an implantable stretchable flexible neural electrode according to an embodiment of the present disclosure;
Fig. 3 is a third plan view of an implantable stretchable flexible neural electrode according to an embodiment of the present disclosure.
Figs. 4a-4f are schematic diagrams of a spiral structure of an implantable stretchable flexible neural electrode according to an embodiment of the present disclosure, respectively;
Fig. 5 is a schematic diagram of an inner circle and an outer circle of a spiral structure of an implantable stretchable flexible neural electrode according to an embodiment of the present disclosure;
Figs. 6a-6d are schematic diagrams of an auxiliary implantation structure of an implantable stretchable flexible neural electrode according to an embodiment of the present disclosure, respectively;
Fig. 7 is a fourth plan view of an implantable stretchable flexible neural electrode according to an embodiment of the present disclosure.
Fig. 8 is a fifth plan view of an implantable stretchable flexible neural electrode according to an embodiment of the present disclosure.
Fig. 9 is a sixth plan view of an implantable stretchable flexible neural electrode according to an embodiment of the present disclosure.
Fig. 10a is a seventh plan view of an implantable stretchable flexible neural electrode according to an embodiment of the present disclosure.
Fig. 10b is an eighth plan view of an implantable stretchable flexible neural electrode according to an embodiment of the present disclosure.
Fig. 11a is a ninth plan view of an implantable stretchable flexible neural electrode according to an embodiment of the present disclosure.
Fig. 11b is a schematic diagram of an anchoring structure of an implantable stretchable flexible neural electrode according to an embodiment of the present disclosure;
Fig. 12 is a tenth plan view of an implantable stretchable flexible neural electrode according to an embodiment of the present disclosure.
Figs. 13a and 13b are schematic cross-sectional views taken along A-A in Fig. 12, respectively.
Figs. 14a-14c are schematic cross-sectional views of an implantable stretchable flexible neural electrode according to an embodiment of the present disclosure, respectively;
Fig. 15 is a first plan view of an implantable stretchable flexible neural electrode group according to an embodiment of the present disclosure;
Figs. 16a-16c are enlarged views of part A in Fig. 15;
Fig. 17a is a second plan view of an implantable stretchable flexible neural electrode group according to an embodiment of the present disclosure;
Fig. 17b is an enlarged view of part B in Fig. 17a;
Fig. 18 is a third plan view of an implantable stretchable flexible neural electrode group according to an embodiment of the present disclosure.
Fig. 19 is a plan view of an implantable stretchable flexible neural electrode array according to an embodiment of the present disclosure;
Fig. 20 is a flowchart of a manufacturing method of an implantable stretchable flexible neural electrode according to an embodiment of the present disclosure;
Fig. 21 is a schematic sectional view of various steps in a manufacturing method of an implantable stretchable flexible neural electrode according to an embodiment of the present disclosure;
Fig. 22 is an exploded view of an implantable stretchable flexible neural electrode according to an embodiment of the present disclosure;
Fig. 23 is an exploded view of an implantable stretchable flexible neural electrode array according to an embodiment of the present disclosure;
Fig. 24 is a flowchart of an implantation method of an implantable stretchable flexible neural electrode according to an embodiment of the present disclosure; and
Figs. 25a-25c are perspective views of various steps in an implantation method of an implantable stretchable flexible neural electrode according to an embodiment of the present disclosure.

### DETAILED DESCRIPTION

In order to make objects, technical solutions and advantages of the embodiments of the present disclosure more apparent, the technical solutions of the embodiments of the present disclosure will be described in a clearly and fully understandable way in connection with the drawings related to the embodiments of the present disclosure. Apparently, the described embodiments are just a part but not all of the embodiments of the present disclosure. Based on the described embodiments herein, those ordinary skilled in the art can obtain other embodiment(s), without any inventive work, which all should be within the scope of protection of the present disclosure.

Unless otherwise defined, all the technical and scientific terms used herein have the same meanings as commonly understood by one of ordinary skill in the art to which the present disclosure belongs. The terms "first," "second," etc., which are used in the present disclosure, are not intended to indicate any sequence, amount or importance, but distinguish various components. Also, the terms "comprise," "comprising," "include," "including," etc., are intended to specify that the elements or the objects stated before these terms encompass the elements or the objects and equivalents thereof listed after these terms, but do not preclude the other elements or objects. "Inner," "outer," "above," "under" and the like are only used to indicate relative position relationship, and when the position of the object which is described is changed, the relative position relationship may be changed accordingly.

The drawings in the present disclosure are not drawn strictly to actual scale, and the specific size and quantity of each structure can be determined according to actual needs. The drawings described in the present disclosure are only structural schematic diagrams.

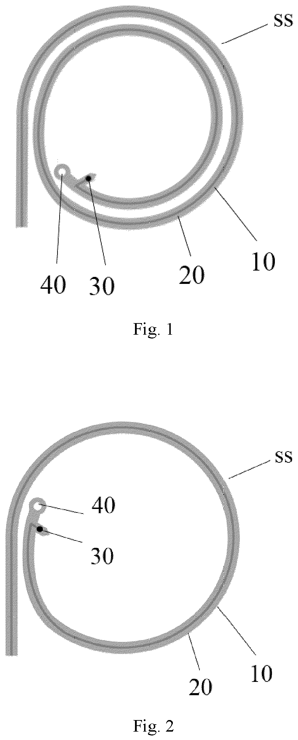

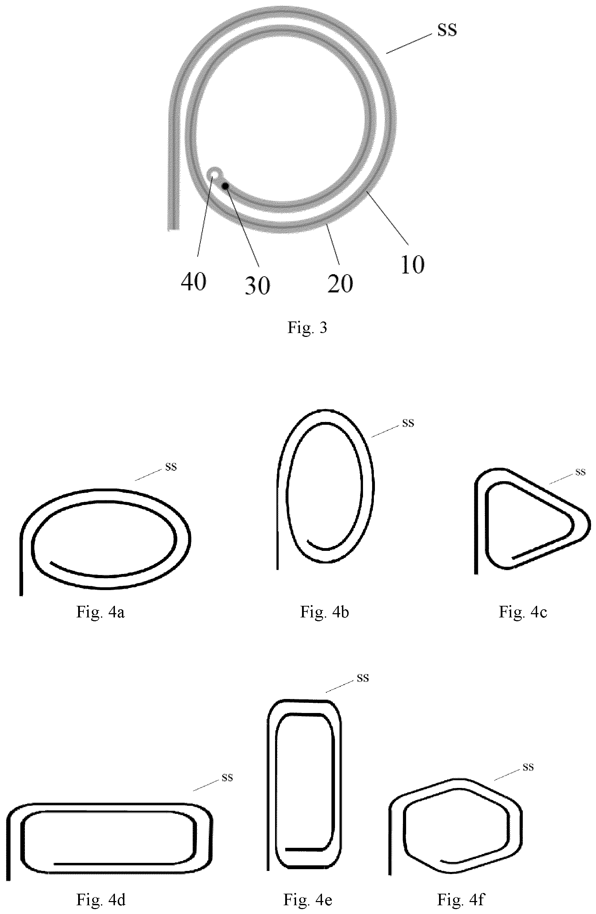

Embodiments of the present disclosure provide an implantable stretchable flexible neural electrode. Fig. 1 is a first plan view of an implantable stretchable flexible neural electrode according to an embodiment of the present disclosure; Fig. 2 is a second plan view of an implantable stretchable flexible neural electrode according to an embodiment of the present disclosure; Fig. 3 is a third plan view of an implantable stretchable flexible neural electrode according to an embodiment of the present disclosure. Referring to Figs. 1-3, an implantable stretchable flexible neural electrode according to an embodiment of the present disclosure includes an electrode wire 10 and a flexible insulating part 20 wrapping the electrode wire 10; the implantable stretchable flexible neural electrode according to the embodiment of the present disclosure further includes a spiral structure SS of the electrode wire 10 and the flexible insulating part 20, and the spiral structure SS is stretchable; an electrode site 30 and an auxiliary implantation structure 40 are provided at the spiral structure SS, and the electrode site 30 is electrically connected to the electrode wire 10 and exposed from the flexible insulating part 20, and the auxiliary implantation structure 40 is configured to drive the spiral structure SS to stretch under the action of an external force.

According to an embodiment of the present disclosure, the flexible neural electrode includes a spiral structure SS of the electrode wire 10 and the flexible insulating part 20, and the spiral structure SS is stretchable. On the one hand, the spiral structure SS can greatly increase the implantation depth of flexible neural electrodes in target tissues (for example, brain tissues); for example, in a case where the spiral structure has a circle shape by way of example, the implantation depth of a two-circle spiral structure with a diameter of 1 mm can reach about 6 mm, and the implantation depth of a four-circle spiral structure with a diameter of 1 mm can reach about 12 mm; for another example, still in the case where the spiral structure has a circle shape by way of example, the implantation depth of a two-circle spiral structure with a diameter of 3 mm can reach about 12 mm, and the implantation depth of a four-circle spiral structure with a diameter of 3 mm can reach about 25 mm; therefore, designing the flexible neural electrode into a spiral structure SS including the electrode wire 10 and the flexible insulating part 20 can greatly improve the realizable length of the flexible neural electrode in the effective area. On another hand, the spiral structure SS has good stretchability, which can realize large-scale stretching in different directions and distances, thus improving the operational flexibility of implanting the flexible neural electrode into the target tissue. On still another hand, the spiral structure SS has good mechanical stability, and can still ensure good structural stability when it is stretched over a large range to be transformed from a planar spiral structure into a three-dimensional curve, which ensures the stability of the flexible neural electrode after being implanted into the target tissue.

For example, the spiral structure SS according to the embodiment of the present disclosure is a regular or irregular looped structure in which any straight line and any curve are combined. Therefore, the spiral structure SS according to the embodiment of the present disclosure includes at least one circle of electrode wire 10 and flexible insulating part 20. For example, the number of circles of the electrode wire 10 and the flexible insulating part 20 in the spiral structure SS is 1 to 100,000 circles, such as, 1 circle, 10 circles, 50 circles, 100 circles, 300 circles, 500 circles, 700 circles or 1,000 circles, and so on. Further, in order to improve the realizable length of the flexible neural electrode in the effective area, for example, the number of circles of the electrode wire 10 and the flexible insulating part 20 in the spiral structure SS is more than 1 circle, such as more than 1 circle, 2 circles, 10 circles, 50 circles, 100 circles, 300 circles, 500 circles, 700 circles or 1000 circles, and so on. As an example, Figs. 1 and 3 show that the spiral structure SS includes two circles of electrode wire 10 and flexible insulating part 20, and Fig. 2 shows that the spiral structure SS includes more than one circle of electrode wire 10 and flexible insulating part 20.

For example, according to an embodiment of the present disclosure, the spiral structure SS is stretchable; further, for example, the spiral structure SS is configured to be stretchable in any direction. In this way, not only the realizable length of the flexible neural electrode in the effective area is improved, but also the flexibility of operation of implanting the flexible neural electrode into the target tissue is improved. For example, "the spiral structure SS is configured to be stretchable in any direction" includes both the case of stretching in a direction in a plane where the spiral structure SS is located and the case of stretching in any direction different from the direction in the plane where the spiral structure SS is located.

As described above, the spiral structure SS according to the embodiment of the present disclosure is a regular or irregular looped structure in which any straight line and any curve are combined. Referring to Figs. 1 to 3, one circle of the spiral structure SS is generally circular; however, the embodiment of the present disclosure does not limit the shape of the one circle of the spiral structure SS. For example, one circle of the spiral structure SS can be generally circular, elliptical, triangular, square, rectangular or arbitrary polygon; one circle of the spiral structure SS can also be generally a rounded triangle, a rounded square, a rounded rectangle or any rounded polygon. In contrast, the machining accuracy of rounded polygons is less than that of normal polygons. Figs. 4a-4f are schematic diagrams of spiral structures of implantable stretchable flexible neural electrodes according to embodiments of the present disclosure, respectively. As an example, Figs. 4a and 4b show that one circle of the spiral structure SS is generally elliptical, and the difference between Figs. 4a and 4b is that the direction of the long axis of the ellipse in Fig. 4a is different from that of the ellipse in Fig. 4b. As an example, Figs. 4d and 4e show that one circle of the spiral structure SS is generally a rounded rectangle, and the difference between Figs. 4d and 4e is that the direction of the long side of the rounded rectangle in Fig. 4d is different from that of the rounded rectangle in Fig. 4e. As an example, Fig. 4c shows that one circle of the spiral structure SS is generally a rounded triangle, while Fig. 4f shows that one circle of the spiral structure is generally a rounded hexagon. It should be noted that, for simplifying the illustration, the electrode wire 10 and the flexible insulating part 20 are not specifically shown in Figs. 4a to 4f, but only the spiral pattern of their spiral structure SS is shown.

For example, according to the embodiment of the present disclosure, the maximum size of the spiral structure SS is 0.1 mm to 2 cm, such as 1 mm, 3 mm, 5 mm, 7 mm, 1 cm or 2 cm, and so on. If the overall size of the spiral structure SS in a certain direction is larger than all the sizes in other directions, the size of the spiral structure SS in the certain direction is the maximum size of the spiral structure SS. For example, in Figs. 4a and 4b, the maximum size of the spiral structure SS is the size of the long axis of ellipse at the outermost circle, which is 0.1 mm to 2 cm.

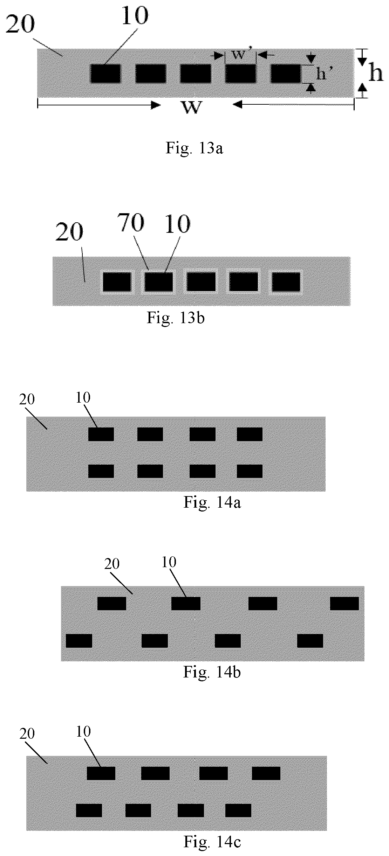

For example, according to the embodiment of the present disclosure, for the spiral structure SS, the height of the cross section of one circle thereof (for example, referring to h in Fig. 13a) is 1 µm to 200 µm, preferably 2 µm; the width (for example, referring to w in Fig. 13a) of the cross section of one circle thereof is 1 µm to 2,000 µm, preferably 20 µm.

For example, according to the embodiment of the present disclosure, it can be understood that the flexible insulating part 20 wrapping the electrode wire 10 means that the flexible insulating part 20 covers all the surfaces of the electrode wire 10 except the surface part of the electrode wire 10 that is in contact with other members.

For example, according to the embodiment of the present disclosure, the flexible insulating part 20 is made of a flexible material with good biocompatibility and mechanical elasticity. Further, for example, the material of the flexible insulating part 20 includes at least one of polyimide (PI), Poly-p-xylene (Parylene C) and SU-8 photoresist. For example, the material of the flexible insulating part 20 includes a combination of SU-8 photoresist and Parylene C, a combination of Parylene C and PI, or a combination of SU-8 photoresist, Parylene C and PI, etc. It is further preferable that the material of the flexible insulating part 20 is PI. For example, the flexible insulating part 20 is transparent or translucent or opaque.

For example, according to the embodiment of the present disclosure, the thickness of the flexible insulating part 20 is 1 µm to 200 µm, for example, 1 µm, 2 µm, 3 µm, 4 µm, 5 µm, 7 µm, 10 µm, 13 µm, 15 µm, 17 µm, 20 µm or 50 µm, etc.; further preferably, 2 µm.

For example, according to an embodiment of the present disclosure, the thickness of the electrode wire 10 (for example, referring to h' in Fig. 13a) is 10 nm to 1000 nm, for example, 10 nm, 50 nm, 100 nm, 300 nm, 500 nm, 800 nm or 1000 nm, etc.; further preferably, 100 nm.

For example, according to the embodiment of the present disclosure, the width of the electrode wire 10 (for example, referring to w' in Fig. 13a) is 1 µm to 200 µm, for example, 1 µm, 5 µm, 10 µm, 50 µm, 100 µm or 200 µm, etc.; further preferably, 20 µm.

For example, according to the embodiment of the present disclosure, "the electrode site 30 and the auxiliary implantation structure 40 are provided at the spiral structure SS" means that the electrode site 30 and the auxiliary implantation structure 40 are provided on and/or connected to at least one circle of the spiral structure SS.

For example, according to the embodiment of the present disclosure, the electrode site 30 is electrically connected to the electrode wire 10 and exposed from the flexible insulating part 20. In this case, after the flexible neural electrode is implanted into the target tissue (for example, brain tissue), the electrode site 30 collects biological information in the target tissue, and the biological information is transmitted to an external circuit via the electrode wire 10 electrically connected to the electrode site 30; and/or, after the flexible neural electrode is implanted into a target tissue (for example, brain tissue), an external circuit applies electric regulation information, which is transmitted to the electrode site 30 electrically connected to the electrode wire 10 via the electrode wire 10, and the electrode site 30 applies the electric regulation information to the target tissue. The electrode site 30 is exposed from the flexible insulating part 20, which can be understood as "the flexible insulating part 20 does not cover at least part of the electrode site 30".

For example, according to the embodiment of the present disclosure, the electrode wire 10 and the electrode site 30 electrically connected to each other are formed of the same material or different materials, but the embodiment of the present disclosure does not limit thereto. For example, in order to simplify the manufacturing process, the electrode wire 10 and the electrode site 30 electrically connected to each other are formed of the same material. For example, according to an embodiment of the present disclosure, the material of the electrode wire 10 and the electrode site 30 includes at least one of gold, platinum and iridium. For example, the material of the electrode wire 10 and the electrode site 30 is a combination of gold and platinum, a combination of platinum and iridium, a combination of gold, platinum and iridium, or the like. It is further preferable that the material of the electrode wire 10 and the electrode site 30 is gold. It should be noted that "the material of the electrode wire 10 and the electrode site 30 includes at least one of gold, platinum and iridium" means that at least the material of the outer surfaces of the electrode wire 10 and the electrode site 30 includes at least one of gold, platinum and iridium.

For example, according to the embodiment of the present disclosure, the shape of the electrode site 30 is a semicircle, a large secant of a circle, a small secant of a circle, an ellipse or a circle, etc.; further preferably, a circle, as shown in Figs. 1-3. For example, the diameter of the circle is 1 µm to 100 µm, for example, 1 µm, 5 µm, 10 µm, 30 µm, 50 µm, 70 µm or 100 µm, etc.; further preferably, 20 µm.

For example, according to the embodiment of the present disclosure, the arrangement of the electrode site 30 is not particularly limited as long as the electrode site 30 and the electrode wire 10 are electrically connected to each other. For example, as shown in Figs. 1 and 2, the electrode site 30 is located outside the electrode wire 10, and is electrically connected to the electrode wire 10 through a connection structure (for example, an anchoring electrode wire 62 as described below). For example, as shown in Fig. 3, the electrode site 30 is located on the electrode wire 10, that is, it is a part of the electrode wire 10; in this case, a part of the electrode wire 10 is exposed from the flexible insulating part 20 to serve as an electrode site 30.

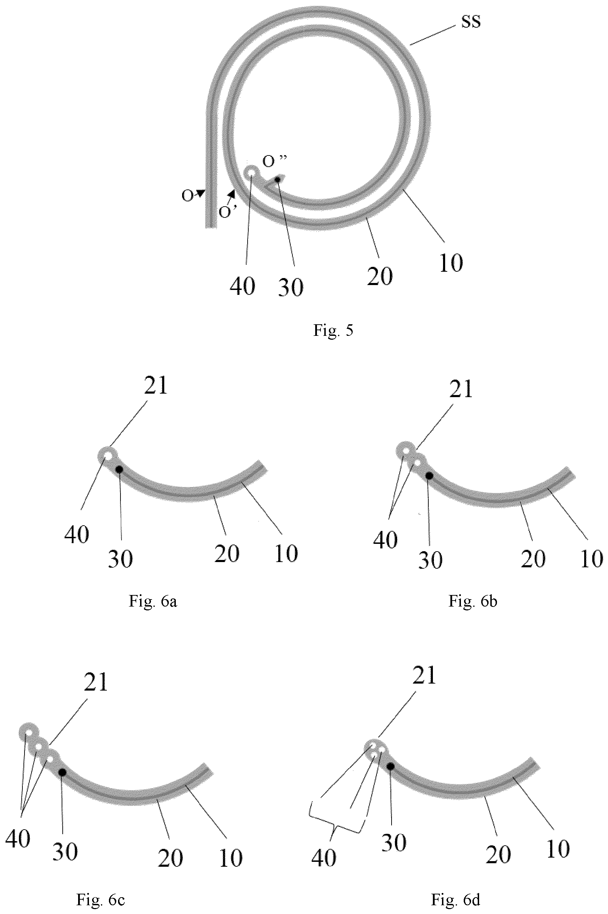

For example, according to the embodiment of the present disclosure, the spiral structure SS includes a plurality of circles, and any two adjacent circles among the plurality of circles are referred to as an inner circle and an outer circle, respectively; wherein the outer circle surrounds the inner circle, and a termination end of the outer circle is connected to an initial end of the inner circle. In this way, since the spiral structure SS includes a plurality of circles, the realizable length of the flexible neural electrode in the effective area can be increased; and since the outer circle surrounds the inner circle and the termination end of the outer circle is connected to the initial end of the inner circle, the plurality of circles are arranged more orderly without being intersected with each other, so that the spiral structure SS can be conveniently manufactured by a patterning process, for example, photolithography and that the stretching process of the spiral structure SS is smoother. Fig. 5 is a schematic diagram of an inner circle and an outer circle of a spiral structure of an implantable stretchable flexible neural electrode according to an embodiment of the present disclosure. Referring to Fig. 5, the part from point O to point O' is an outer circle, and the part from point O' to point O" is an inner circle. The outer circle OO' surrounds the inner circle O'O", and the termination end of the outer circle OO' is connected to the initial end of the inner circle O'O". In Fig. 5, the termination end of the outer circle OO' and the initial end of the inner circle O'O" are indicated by the point O'.

It should be noted that in the already described Fig.1, Fig.3, Figs. 4a-4f, and Fig. 5, the plurality of circles of the spiral structure SS are basically a plurality of circles with the same shape. However, embodiments of the present disclosure are not limited thereto; the shapes of at least two circles of the spiral structure SS can be different from each other, which can be flexibly designed according to the actual situation.

It should be noted that in the already described Fig.1, Fig.3, Figs. 4a-4f, and Fig. 5, the plurality of circles of the spiral structure SS are basically arranged at equal intervals. However, embodiments of the present disclosure are not limited thereto; the plurality of circles of the spiral structure SS can be arranged at different intervals, which can be flexibly designed according to the actual situation.

It should be noted that in the already described Fig.1, Fig.3, Figs. 4a-4f, and Fig. 5, the distance between the inner circle and the outer circle of the spiral structure SS is equal everywhere. However, embodiments of the present disclosure are not limited thereto; the distance between the inner circle and the outer circle of the spiral structure SS can be varied, which can also be designed flexibly according to the actual situation.

For example, according to the embodiment of the present disclosure, the auxiliary implantation structure 40 is configured to drive the spiral structure SS to stretch under the action of an external force. That is, according to the embodiment of the present disclosure, on the one hand, the auxiliary implantation structure 40 has a function of assisting implantation, and can drive at least part of the flexible neural electrode to be implanted into the target tissue; and on the other hand, the auxiliary implantation structure 40 can also drive at least part of the spiral structure SS to stretch. For example, in order for the auxiliary implant structure 40 to better realize its two functions as described above, the auxiliary implant structure 40 is provided at the innermost circle among the plurality of circles of the spiral structure SS, that is, provided on and/or connected to the innermost circle. Further, for example, the auxiliary implantation structure 40 is provided at the termination end of the innermost circle among the plurality of circles of the spiral structure SS, so as to better realize the functions of assisting implantation and stretching the spiral structure SS. It should be noted that the arrangement position of the electrode site 30 is more flexible than that of the auxiliary implantation structure 40, and the electrode site 30 can be provided at the innermost circle and/or other circles of the spiral structure SS.

For example, according to the embodiment of the present disclosure, the flexible insulating part 20 has a protruding part 21 extending beyond the electrode wire 10, and the auxiliary implantation structure 40 is a through hole, a groove or a protrusion provided at the protruding part 21. For example, in the case where the auxiliary implantation structure 40 is a through hole or a groove, an auxiliary implantation instrument cooperating with the auxiliary implantation structure 40 may have a protrusion engaged with the through hole or the groove; in the case where the auxiliary implantation structure 40 is a protrusion, the auxiliary implantation instrument cooperating with the auxiliary implantation structure 40 may have a hole or a recess engaged with the protrusion. Figs. 6a-6d are schematic diagrams of the auxiliary implantation structure of the implantable stretchable flexible neural electrode according to the embodiment of the present disclosure, respectively. As an example, Figs. 6a-6d show the auxiliary implant structure 40 as a through hole. In addition, the embodiment of the present disclosure does not limit the number of the auxiliary implant structure 40, for example, the number of the auxiliary implant structure 40 may be one or more. In the case of a plurality of auxiliary implant structures 40, when one auxiliary implant structure 40 fails, other auxiliary implant structures 40 can be utilized, thus greatly improving the operation friendliness and implantation feasibility of the flexible neural electrode according to the embodiment of the present disclosure. By way of example, Figs. 6b-6d show a plurality of auxiliary implantation structures 40. It should be noted that in the case of a plurality of auxiliary implant structures 40, the arrangement of the plurality of auxiliary implant structures 40 is not specifically limited in the embodiment of the present disclosure, and can be flexibly designed according to the situation. As an example, Fig. 6c shows one arrangement mode of three auxiliary implant structures 40, and Fig. 6d shows another arrangement mode of three auxiliary implant structures 40.

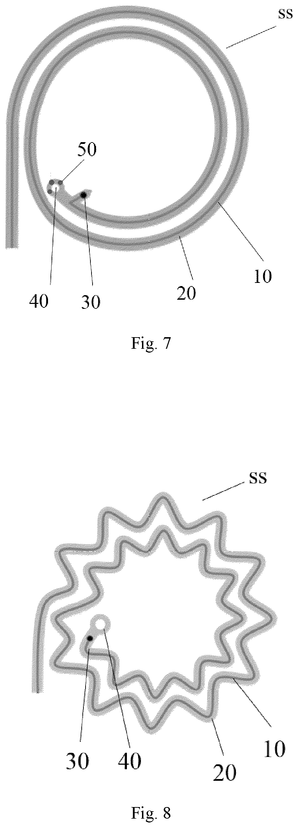

Fig. 7 is a fourth plan view of an implantable stretchable flexible neural electrode according to an embodiment of the present disclosure. Referring to Fig. 7, for example, a position mark 50 is provided on the protruding part 21. By setting the position mark 50, the position where the flexible neural electrode according to the embodiment of the present disclosure is implanted into the target tissue can be conveniently determined. Further, for example, the position mark 50 is provided at the same layer as the electrode wire 10 and made of the same material as the electrode wire 10, so that the position mark 50 and the electrode wire 10 can be formed in the same patterning process, and the manufacturing process is simplified. In addition, it should be noted that the position mark 50 is not limited to be provided at the protruding part 21, but may be provided at any required position according to the situation.

Fig. 8 is a fifth plan view of an implantable stretchable flexible neural electrode according to an embodiment of the present disclosure. For example, referring to Fig. 8, each circle of a plurality of circles of the spiral structure SS includes a plurality of curved portions connected in sequence, thereby further improving the achievable length of the flexible neural electrode in the effective area. It should be noted that the plurality of circles of the spiral structure SS may include the same or different curved portions, and only some circles of the spiral structure SS may include the curved portions, and the embodiment of the present disclosure does not limit thereto.

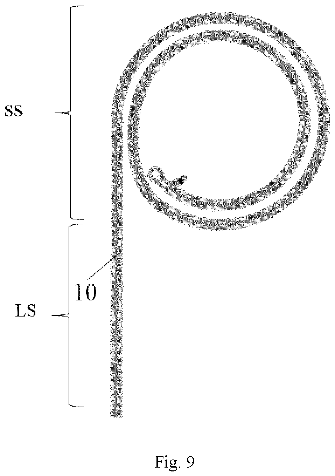

For example, the flexible neural electrode according to the embodiment of the present disclosure may include only the spiral structure SS, and may also include other structures besides the spiral structure SS. Fig. 9 is a sixth plan view of an implantable stretchable flexible neural electrode according to an embodiment of the present disclosure. For example, referring to Fig. 9, the flexible neural electrode according to the embodiment of the present disclosure further includes a linear structure LS of the electrode wire 10 and the flexible insulating part 20, and the linear structure LS is connected to the spiral structure SS. The flexible neural electrode including the linear structure LS may make the connection between the flexible neural electrode and the external circuit more convenient. It should be noted that according to the embodiment of the present disclosure, the linear structure SS does not necessarily have to be a straight line, as long as its size in one direction is much greater than that in other directions; therefore, according to the embodiment of the present disclosure, the linear structure SS can be a straight line or a curve such as a broken line or a wavy line, which can be flexibly designed according to the situation. In addition, as an example, Fig. 9 shows that a straight angle exists between the linear structure LS and the initial end of the outer circle of the spiral structure SS; however, the embodiment of the present disclosure is not intended to make any limitation thereto, and any angle may exist between the linear structure LS and the initial end of the outer circle of the spiral structure SS.

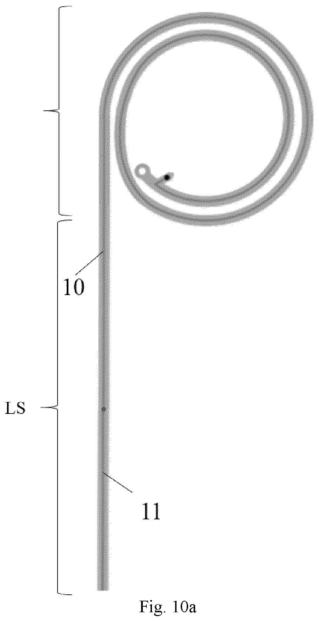

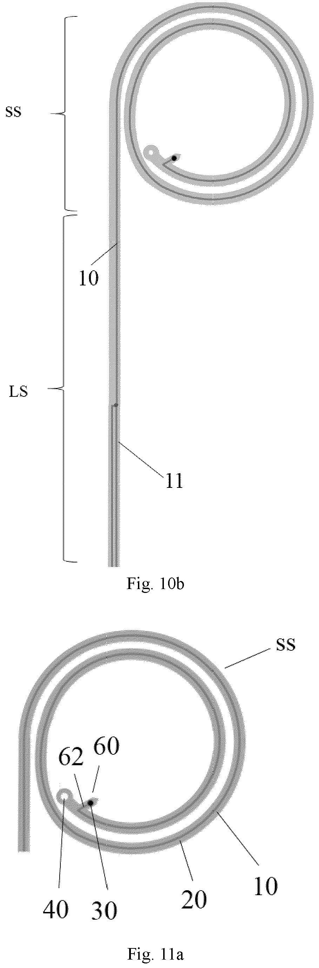

Fig. 10a is a seventh plan view of an implantable stretchable flexible neural electrode according to an embodiment of the present disclosure; Fig. 10b is an eighth plan view of an implantable stretchable flexible neural electrode according to an embodiment of the present disclosure. Referring to Figs. 10a and 10b, the linear structure LS further includes an auxiliary electrode wire 11 wrapped by the flexible insulating part 20; and in the linear structure LS, the auxiliary electrode wire 11 is electrically connected to the electrode wire 10. By arranging the auxiliary electrode wire 11, the wiring length of the electrode wire 10 can be extended, thereby further improving the wiring flexibility of the flexible neural electrode according to the embodiment of the present disclosure. For example, the electrode wire 10 and the auxiliary electrode wire 11 may be provided at the same layer or at different layers, may be formed of the same material or different materials, and may have the same size or different sizes, which is not limited in the embodiment of the present disclosure. For example, the length of the auxiliary electrode wire 11 is 1 mm to 10 cm, for example, 1 mm, 3 mm, 5 mm, 7 mm, 1 cm or 5 cm, etc.; further preferably, 1 cm or 2.5 cm. For example, the width of the auxiliary electrode wire 11 is 1 µm to 50 µm, for example, 1 µm, 5 µm, 10 µm, 20 µm, 30 µm, 40 µm or 50 µm, etc.; further preferably, 1.5 µm.

For example, referring to Fig. 10b, the linear structure LS includes a plurality of auxiliary electrode wires 11 wrapped by the flexible insulating part 20, wherein the plurality of auxiliary electrode wires 11 are insulated from each other and electrically connected to the electrode wire 10, respectively. For example, in the case where a plurality of auxiliary electrode wires 11 are provided, when one auxiliary electrode wire 11 fails, another auxiliary electrode wire 11 can be utilized, thereby enhancing the reliability of the flexible neural electrode according to the embodiment of the present disclosure.

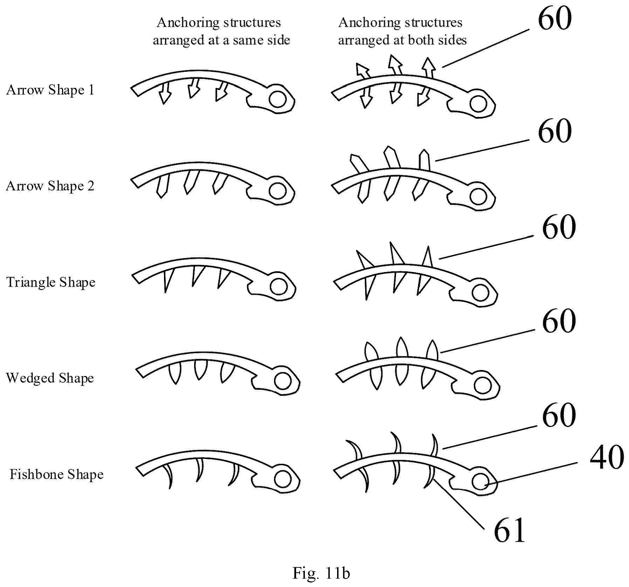

Fig. 11a is a ninth plan view of an implantable stretchable flexible neural electrode according to an embodiment of the present disclosure. For example, referring to Fig. 11a, the implantable stretchable flexible neural electrode according to the embodiment of the present disclosure further includes an anchoring structure 60; there is an angle between the anchoring structure 60 and the electrode wire 10, and the anchoring structure 60 is configured to be interacted with the target tissue after the flexible neural electrode is implanted into the target tissue so as to prevent the flexible neural electrode from moving relative to the target tissue. By arranging the anchoring structure 60, the part of the flexible neural electrode implanted into the target tissue can be more stably maintained in the target tissue, so that the flexible neural electrode can stably detect or regulate the target tissue. For example, there is an angle between the anchoring structure 60 and the electrode wire 10, and the angle can be any angle as long as the anchoring structure 60 interacts with the target tissue to prevent the flexible neural electrode from moving relative to the target tissue. For example, the anchoring structure 60 protrudes from the flexible insulating part 20 that wraps the electrode wire 10. For example, the number of the anchoring structures 60 is 1-20, such as 1, 4, 8, 16 or 20, which is not limited in the embodiment of the present disclosure. For example, in order to enhance the anchoring effect, it is preferable that a plurality of anchoring structures 60 are provided; in this case, the plurality of anchoring structures 60 may be located at the same side or different sides of the electrode wire 10, and the shapes of the plurality of anchoring structures 60 may be the same or different, which is not limited in the embodiment of the present disclosure. For one anchoring structure 60, along a direction away from the electrode wire 10, the anchoring structure 60 may change from wide to narrow, from narrow to wide, or sometimes narrow and sometimes wide, or maintain constant width, which is not limited in the embodiment of the present disclosure. Fig. 11b is a schematic diagram of an anchoring structure of an implantable stretchable flexible neural electrode according to an embodiment of the present disclosure. As an example, Fig. 11b shows that the anchoring structure 60 can be an arrow-shaped structure with a thickened end (see arrow shape 1, for example), an arrow-shaped structure with a tapered end (see arrow shape 2, for example), a triangular structure (see the triangular shape, for example), a wedge-shaped structure (see the wedged shape, for example), or a fishbone-shaped structure (see the fishbone shape, for example). However, the embodiment of the present disclosure is not limited thereto, and the shape of the anchoring structure 60 can be flexibly designed as required, as long as the anchoring structure 60 interacts with the target tissue after the flexible neural electrode is implanted into the target tissue to prevent the flexible neural electrode from moving relative to the target tissue. In Fig. 11b, the left column shows a plurality of anchoring structures 60 located at the same side of the electrode wire 10, while the right column shows a plurality of anchoring structures 60 located at both sides of the electrode wire 10, respectively. In the case where the plurality of anchoring structures 60 are located at both sides of the electrode wire 10, respectively, the number of the anchoring structures 60 located at both sides of the electrode wire 10 may be equal or unequal, which is not limited in the embodiment of the present disclosure.

For example, referring to the structure in the last row of Fig. 11b, the edge of the anchoring structure 60 extending from the electrode wire 10 in a direction away from the electrode wire 10 is an arc shape 61, and the arc shape 61 protrudes towards the auxiliary implantation structure 40. By arranging the arc shape 61 protruding towards the auxiliary implantation structure 40, the implantation process of the flexible neural electrode according to the embodiment of the present disclosure can be made smoother.

For example, with continued reference to Fig. 11a, the anchoring structure 60 includes an anchoring electrode wire 62 covered by the flexible insulating part 20, and the electrode site 30 is electrically connected to the electrode wire 10 through the anchoring electrode wire 62. In this way, there is no need to provide a specialized connection structure for the electrode wire 10 and the electrode site 30, which allows the structure of the flexible neural electrode according to the embodiment of the present disclosure to be simpler and easier to realize. However, it should be noted that the embodiment of the present disclosure is not limited thereto, and the anchoring structure 60 may be only a protrusion of the flexible insulating part 20 without including the anchoring electrode wire 62.

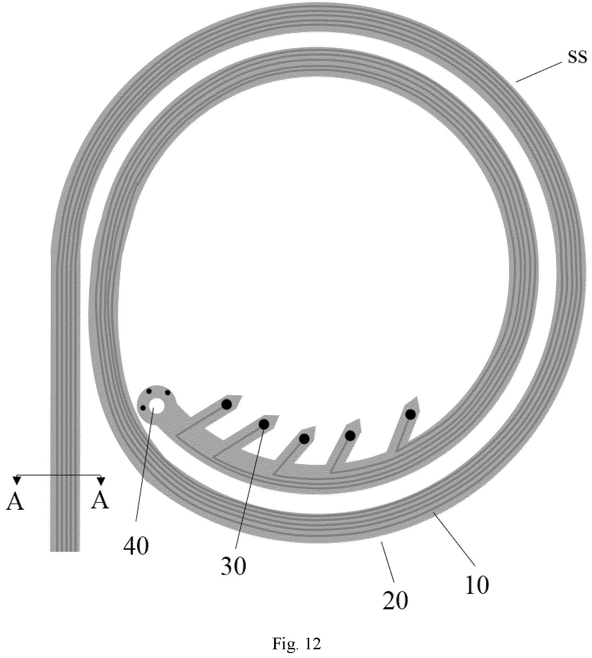

Fig. 12 is a tenth plan view of an implantable stretchable flexible neural electrode according to an embodiment of the present disclosure. Figs. 13a and 13b are schematic cross-sectional views taken along A-A of Fig. 12, respectively, that is, Figs. 13a and 13b are schematic cross-sectional views of one circle of the spiral structure SS shown in Fig. 12. For example, referring to Fig. 12 and Figs. 13a-13b, a flexible neural electrode according to an embodiment of the present disclosure includes a plurality of electrode wires 10 wrapped by a flexible insulating part 20 and insulated from each other; and a plurality of electrode sites 30 exposed from the flexible insulating part 20, electrically connected to the plurality of electrode wires 10 in one-to-one correspondence, and spatially separated from each other. In the case that the flexible neural electrode according to the embodiment of the present disclosure includes a plurality of electrode wires 10, each circle of the spiral structure SS of the flexible neural electrode includes the plurality of electrode wires 10. For example, the number of the plurality of electrode wires 10 is 1 to 10000, for example, 1, 10, 50, 100, 300, 500, 700 or 1000, etc., and more preferably, 10; accordingly, the number of the plurality of electrode sites is 1 to 10000, for example, 1, 10, 50, 100, 300, 500, 700 or 1000, etc., and more preferably, 10. After the flexible neural electrode according to the embodiment of the present disclosure is implanted into a target tissue (for example, brain tissue), the plurality of electrode sites 30 can monitor and/or regulate a plurality of positions of the target tissue, thereby greatly improving the monitoring and/or regulating effect of the flexible neural electrode according to the embodiment of the present disclosure. For example, the plurality of electrode sites 30 are spatially separated from each other by about 70 µm. As an example, Figs. 12, 13a and 13b show 5 electrode wires 10 and 5 electrode sites 30.

For example, referring to Fig. 13b, the implantable stretchable flexible neural electrode according to the embodiment of the present disclosure further includes an adhesive layer 70 provided between the electrode wire 10 and the flexible insulating part 20. By arranging the adhesive layer 70, the bonding strength between the electrode wire 10 and the flexible insulating part 20 can be enhanced, and the structure of the flexible neural electrode is more stable. For example, the material of the adhesive layer 70 includes chromium or titanium; further preferably, chromium. For example, the thickness of the adhesive layer 70 is 1 nm to 100 nm, for example, 1 nm, 5 nm, 10 nm, 20 nm, 30 nm, 40 nm, 50 nm, 60 nm, 70 nm, 80 nm, 90 nm or 100 nm; further preferably, 5 nm.

In Figs. 13a and 13b, the plurality of electrode wires 10 are located at the same layer. However, the embodiment of the present disclosure is not limited thereto. The flexible neural electrode according to the embodiment of the present disclosure includes a plurality of electrode wires 10 located at a plurality of layers provided in a stacked manner, and each of the plurality of layers includes at least one of the plurality of electrode wires 10. According to the embodiment of the present disclosure, when the plurality of electrode wires 10 are located at a plurality of stacked layers, each layer may include one electrode wire 10 or a plurality of electrode wires 10, and the number of electrode wires 10 included in each layer may be equal or unequal, which is not limited in the embodiment of the present disclosure. Figs. 14a-14c are schematic cross-sectional views of an implantable stretchable flexible neural electrode according to an embodiment of the present disclosure, respectively. As an example, Figs. 14a-14c show 10 electrode wires 10, of which 5 electrode wires 10 are provided at a lower layer and the other 5 electrode wires are provided at an upper layer. According to an embodiment of the present disclosure, any two adjacent layers among the plurality of layers are referred to as a first layer and a second layer, respectively; in the stacking direction of the plurality of layers, the electrode wire 10 located at the first layer at least partially overlaps or at least partially does not overlap with the electrode wire 10 located at the second layer. As an example, Fig. 14a shows that the electrode wire 10 located at the first layer completely overlaps with the electrode wire 10 located at the second layer in the stacking direction; Fig. 14b shows that the electrode wire 10 located at the first layer does not completely overlap with the electrode wire 10 located at the second layer in the stacking direction; and Fig. 14c shows that the electrode wire 10 located at the first layer partially overlaps and partially does not overlap with the electrode wire 10 located at the second layer in the stacking direction. For example, the electrode wire 10 located at the first layer and the electrode wire 10 located at the second layer are implanted into the target tissue simultaneously. For example, the electrode wire 10 located at the first layer and the electrode wire 10 located at the second layer are alternately implanted into the target tissue.

Referring to Fig. 12, the plurality of electrode sites 30 are located at the same side of the flexible neural electrode. However, the embodiment of the present disclosure is not limited thereto, and the plurality of the electrode sites 30 may be located at the same side of the flexible neural electrode or located at different sides of the flexible neural electrode. Referring to Fig. 12, the plurality of electrode sites 30 are sequentially arranged, so that the plurality of electrode sites 30 are sequentially implanted into a target tissue at different depths of the target tissue. However, the embodiment of the present disclosure is not limited thereto. The plurality of electrode sites 30 may also be arranged at a plurality of layers so that the plurality of electrode sites 30 are simultaneously implanted into the target tissue and located at different positions at the same depth of the target tissue.

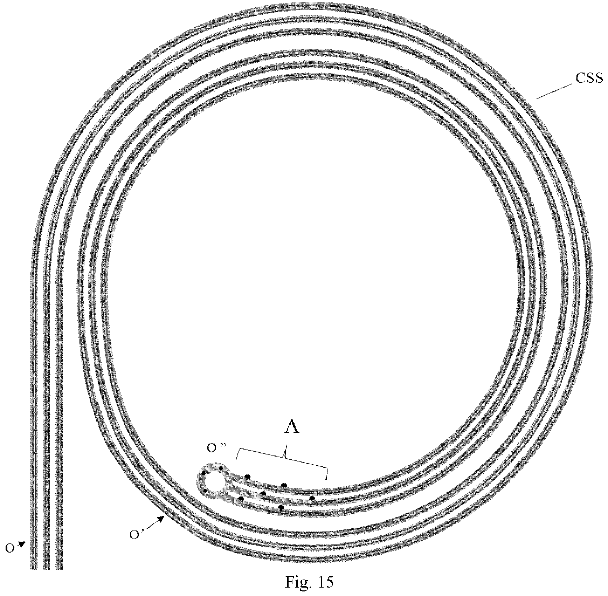

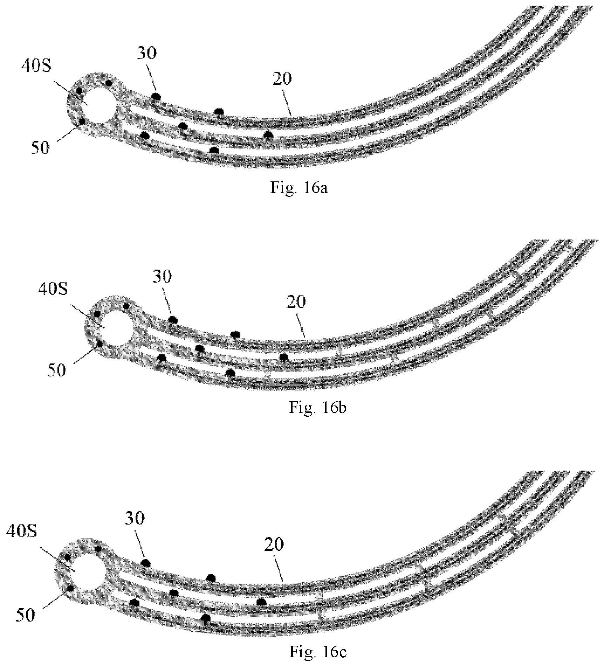

According to the embodiment of the present disclosure, an implantable stretchable flexible neural electrode group is also provided. Fig. 15 is a first plan view of an implantable stretchable flexible neural electrode group according to an embodiment of the present disclosure; and Figs. 16a-16c are enlarged views of part A of Fig. 15. Referring to Fig. 15 and Figs. 16a-16c, an implantable stretchable flexible neural electrode group according to an embodiment of the present disclosure includes a plurality of flexible neural electrodes as described above; the plurality of flexible neural electrodes are insulated from each other; a plurality of spiral structures SS of flexible neural electrodes as described above forms a composite spiral structure CSS, and in a same circle of the composite spiral structure CSS, the parts of the plurality of spiral structures SS located in the same circle are sequentially arranged from inside to outside. For example, a plurality of electrode sites 30 of the plurality of flexible neural electrodes are spatially separated from each other, which can avoid mutual interference between the plurality of electrode sites 30 and is beneficial for the plurality of electrode sites 30 to be dispersed in different positions of the target tissue after being implanted into the target tissue (for example, brain tissue). By providing the flexible neural electrode group, the number of the electrode sites 30 in the effective area is greatly increased, thereby considerably improving the monitoring efficiency and/or regulating efficiency of the flexible neural electrodes according to the embodiment of the present disclosure. According to the embodiment of the present disclosure, the mechanical properties of the flexible neural electrode group are matched with the target tissue (for example, brain tissue) without causing an inflammatory reaction of the target tissue, and the target tissue can be stably monitored and/or regulated at multiple points for a long time. The number of the flexible neural electrodes included in the flexible neural electrode group and the number of the electrode wires 10 included in each flexible neural electrode are not limited in the embodiment of the present disclosure. The arrangement of all the electrode sites included in the flexible neural electrode group is not limited in the embodiment of the present disclosure, and these electrode sites can be provided such that they are implanted into the target tissue sequentially or simultaneously, or a part of these electrode sites can be implanted into the target tissue sequentially and the other part can be implanted into the target tissue simultaneously. As an example, the flexible neural electrode group shown in Figs. 15 and Figs. 16a-16c includes three flexible neural electrodes as described above, and each flexible neural electrode includes two electrode wires 10 and two electrode sites 30. According to the embodiment of the present disclosure, the plurality of flexible neural electrodes are insulated from each other, which means that the electrode wires 10 of the plurality of flexible neural electrodes are insulated from each other, and the plurality of electrode sites 30 of the plurality of flexible neural electrodes are also insulated from each other.

For example, according to the embodiment of the present disclosure, the composite spiral structure CSS is stretchable; further, for example, the composite spiral structure CSS is configured to be stretchable in any direction, so that the flexibility of operation of implanting the flexible neural electrode group into the target tissue is greatly improved. For example, "the composite spiral structure CSS is configured to be stretchable in any direction" includes both the case of stretching in a direction in a plane where the composite spiral structure CSS is located and the case of stretching in any direction different from the direction in the plane where the composite spiral structure CSS is located.

With continued reference to Fig. 15, the composite spiral structure CSS includes a plurality of circles, and any two adjacent circles among the plurality of circles are referred to as an inner circle and an outer circle, respectively, wherein the outer circle surrounds the inner circle, and the termination end of the outer circle is connected to the initial end of the inner circle; in each circle of the plurality of circles of the composite spiral structure CSS, the corresponding parts of the plurality of spiral structures SS are sequentially arranged from inside to outside in the same order. In this way, the plurality of circles of the composite spiral structure CSS are arranged more orderly without being intersected with each other, and the plurality of spiral structures SS of the plurality of flexible neural electrodes are also orderly arranged without being intersected with each other, so that the composite spiral structure CSS can be conveniently manufactured by a patterning process such as photolithography and that the stretching process of the composite spiral structure CSS is smoother. Referring to Fig. 15, the part from point O to point O' is an outer circle, and the part from point O' to point O" is an inner circle. The outer circle OO' surrounds the inner circle O'O", and the termination end of the outer circle OO' is connected to the initial end of the inner circle O'O". In Fig. 15, the termination end of the outer circle OO' and the initial end of the inner circle O'O" are indicated by the point O'.

With continued reference to Fig. 15 and Figs. 16a-16c, the plurality of auxiliary implantation structures 40 of the plurality of flexible neural electrodes included in the flexible neural electrode group are integrated into a common auxiliary structure 40S, which is configured to drive the plurality of spiral structures SS of the plurality of flexible neural electrodes to stretch synchronously under the action of an external force. In this way, the structure of the flexible neural electrode group can be made simpler, and the plurality of flexible neural electrodes included in the flexible neural electrode group can be implanted synchronously, and the plurality of spiral structures SS of the plurality of flexible neural electrodes can be stretched synchronously, thereby improving the implantation efficiency.

Referring to Figs. 16b and 16c, the plurality of flexible insulating parts 20 of the plurality of flexible neural electrodes are at least partially connected to each other, so that the structural stability of the composite spiral structure CSS can be enhanced. The difference between Fig. 16b and Fig. 16c is that the connection positions of the plurality of flexible insulating parts 20 are different, and the embodiment of the present disclosure does not limit the connection positions of the plurality of flexible insulating parts 20.

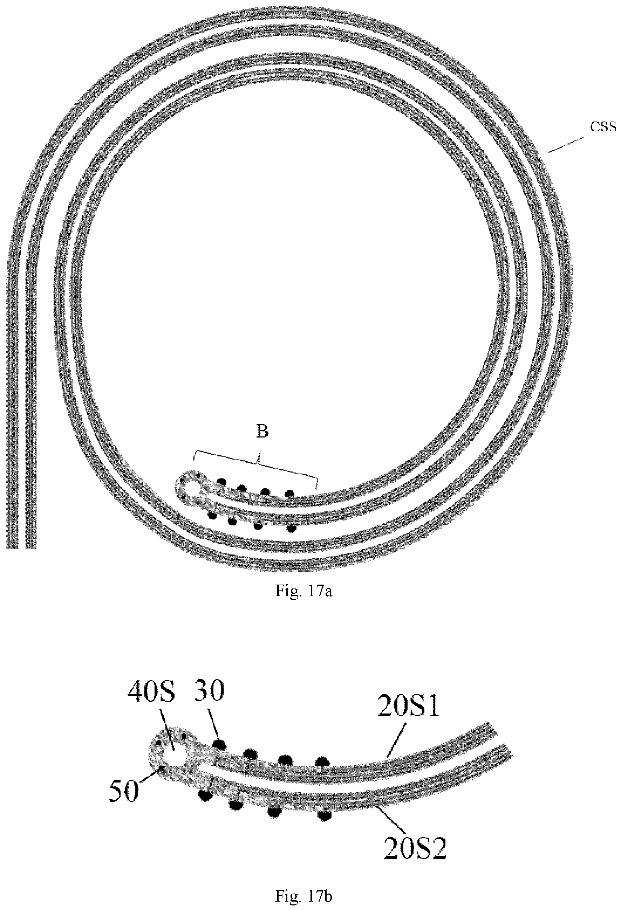

Fig. 17a is a second plan view of an implantable stretchable flexible neural electrode group according to an embodiment of the present disclosure; Fig. 17b is an enlarged view of part B of Fig. 17a. Referring to Figs. 17a and 17b, the plurality of flexible neural electrodes included in the flexible neural electrode group are divided into a first subgroup and a second subgroup; the flexible insulating parts 20 of all the flexible neural electrodes included in the first subgroup are integrated into a first common flexible insulating part 20S1; the flexible insulating parts 20 of all the flexible neural electrodes included in the second subgroup are integrated into a second common flexible insulating part 20S2. In this way, while greatly increasing the number of electrode sites 30 within the effective area, the structure of the flexible neural electrode group is made simpler. For example, the number of the flexible neural electrodes included in the first subgroup and the number of the flexible neural electrodes included in the second subgroup may be the same or different. For example, the number of the electrode wires 10 included in the first subgroup may be the same as or different from the number of the electrode wires 10 included in the second subgroup. As an example, Figs. 17a and 17b show that the first subgroup includes four electrode wires 10 and the second subgroup includes four electrode wires 10. For example, referring to Figs. 17a and 17b, the plurality of electrode sites 30 of the plurality of flexible neural electrodes included in the first subgroup are located at the side of the first subgroup away from the second subgroup, and the plurality of electrode sites 30 of the plurality of flexible neural electrodes included in the second subgroup are located at the side of the second subgroup away from the first subgroup; in this way, interference between the electrode sites 30 can be avoided.

For example, with continued reference to Figs. 17a and 17b, the plurality of auxiliary implantation structures 40 of the plurality of flexible neural electrodes are integrated into a common auxiliary structure 40S, which is configured to drive the spiral structures SS of all the flexible neural electrodes included in the first subgroup and the second subgroup to stretch synchronously under the action of an external force. In this way, the structure of the flexible neural electrode group can be made simpler, and the plurality of flexible neural electrodes included in the flexible neural electrode group can be implanted synchronously, and the plurality of spiral structures SS of the plurality of flexible neural electrodes can be stretched synchronously, thereby improving the implantation efficiency.

For example, as shown in Fig. 17b, the first common flexible insulating part 20S1 and the second common flexible insulating part 20S2 are at least partially connected, so that the structural stability of the composite spiral structure CSS can be enhanced.

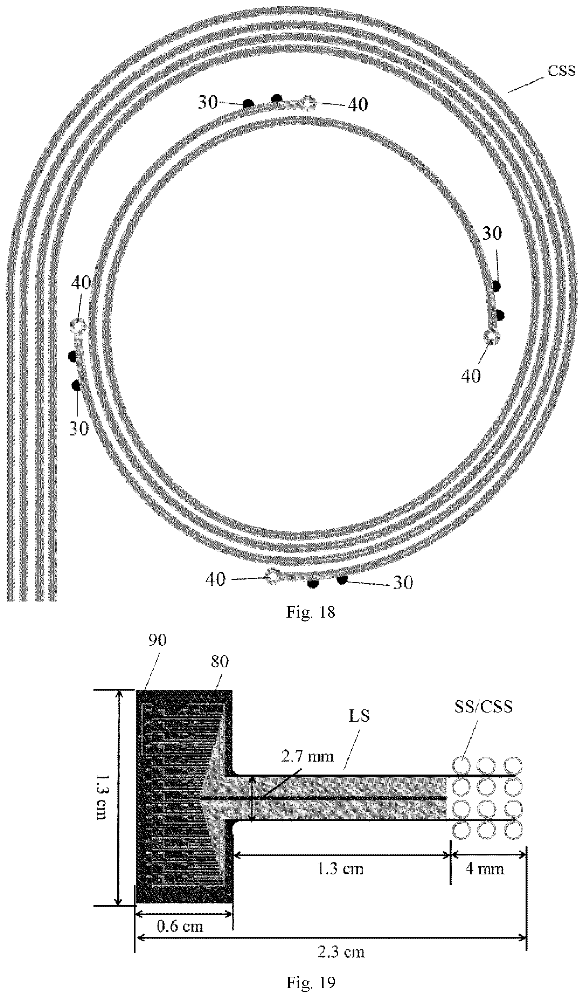

Fig. 18 is a third plan view of an implantable stretchable flexible neural electrode group according to an embodiment of the present disclosure. In the flexible neural electrode group shown in Fig. 18, the plurality of auxiliary implant structures 40 of the plurality of flexible neural electrodes are spatially separated from each other; in this way, multi-point implantation can be realized, so that the implantation position of each flexible neural electrode can be selected more flexibly according to actual needs, and the implantation density can be improved. The embodiment of the present disclosure does not limit the spaced distance between the plurality of auxiliary implantation structures 40, as long as they do not interfere with each other for implantation.

It should be noted that in the implantable stretchable flexible neural electrode group shown in Figs. 15, 17a and 18, only the composite spiral structure CSS is shown for the convenience of illustration. However, the embodiment of the present disclosure is not limited thereto, and the flexible neural electrode group may also include other structures besides the composite spiral structure CSS. For example, each of the plurality of flexible neural electrodes included in the flexible neural electrode group further includes a linear structure of the electrode wire 10 and the flexible insulating part 20. Regarding the design of the linear structure, reference can be made to the previous description, which will not be repeated here.

According to the embodiment of the present disclosure, an implantable stretchable flexible neural electrode array is also provided. Fig. 19 is a plan view of an implantable stretchable flexible neural electrode array according to an embodiment of the present disclosure. Referring to Fig. 19, the flexible neural electrode array according to the embodiment of the present disclosure includes a plurality of flexible neural electrodes as described above, the plurality of flexible neural electrodes are insulated from each other, and the plurality of spiral structures SS of the plurality of flexible neural electrodes are arranged in an array. By adopting the array of flexible neural electrodes, the plurality of spiral structures SS included in the array can be synchronously transferred and implanted, which is convenient to operate and improves the efficiency. According to the embodiment of the present disclosure, each spiral structure SS included in the flexible neural electrode array is stretchable; further, each spiral structure SS included in the flexible neural electrode array is stretchable in any direction. According to the embodiment of the present disclosure, the mechanical properties of the flexible neural electrode array are matched with the target tissue (for example, brain tissue) without causing an inflammatory reaction of the target tissue, so that the target tissue can be stably monitored and/or regulated at multiple points for a long time. According to the embodiment of the present disclosure, the array in which the spiral structures SS are arranged may be a regular array or an irregular array. By comparison, the regular array is more convenient for operation than the irregular array. In addition, the embodiment of the present disclosure does not limit the number of the spiral structures SS included in the array. As an example, Fig. 19 shows that 12 spiral structures SS are arranged in a regular 3x4 array.

With continued reference to Fig. 19, each of the plurality of flexible neural electrodes included in the flexible neural electrode array includes a linear structure LS of the electrode wire 10 and the flexible insulating part 20, and the linear structure LS is connected to the spiral structure SS. In this way, the plurality of flexible neural electrodes included in the flexible neural electrode array can be more conveniently connected to an external circuit 90. Referring to Fig. 19, each electrode wire 10 is connected to a pad 80, and the pad 80, in turn, is connected to the external circuit 90; that is, the electrode wire 10 is connected to the external circuit 90 through the pad 80 connected thereto. It should be noted that the sizes of various parts shown in Fig. 19 are only exemplary, and should not be regarded as limitations on the embodiment of the present disclosure. For example, in order to facilitate the connection with the external circuit 90, the plurality of linear structures LS of the plurality of flexible neural electrodes included in the flexible neural electrode array are located at the same side of an array formed by the spiral structures SS. However, the embodiment of the present disclosure is not limited thereto, and the plurality of linear structures LS of the plurality of flexible neural electrodes included in the flexible neural electrode array can also be located at two opposite sides, two adjacent sides, three sides or multiple sides of an array formed by the spiral structures SS, which can be flexibly designed according to the actual situation.

According to the embodiment of the present disclosure, an implantable stretchable flexible neural electrode array is also provided. With continued reference to Fig. 19, the flexible neural electrode array according to the embodiment of the present disclosure includes a plurality of flexible neural electrode groups as described above, and the plurality of composite spiral structures CSS of the plurality of flexible neural electrode groups are arranged in an array. By adopting the array of flexible neural electrode groups, the plurality of spiral structures SS included in the array can be synchronously transferred and implanted, which is convenient to operate and improves the efficiency; furthermore, the array can include more electrode sites 30, so that the monitoring efficiency and/or regulating efficiency of the technical solution according to the embodiment of the present disclosure is improved. According to the embodiment of the present disclosure, each composite spiral structure CSS included in the flexible neural electrode array is stretchable; further, each composite spiral structure CSS included in the flexible neural electrode array is stretchable in any direction. According to the embodiment of the present disclosure, the array formed by the composite spiral structures CSS can be a regular array or an irregular array. By comparison, the regular array is more convenient for operation than the irregular array. In addition, the embodiment of the present disclosure does not limit the number of the composite spiral structures CSS included in the array. As an example, Fig. 19 shows that 12 composite spiral structures CSS are arranged in a regular 3x4 array.

With continued reference to Fig. 19, each of all the flexible neural electrodes included in the plurality of flexible neural electrode groups includes a linear structure LS of the electrode wire 10 and the flexible insulating part 20, and the linear structure LS is connected to the spiral structure SS. In this way, all the flexible neural electrodes included in the flexible neural electrode array can be connected to an external circuit 90 more conveniently. Referring to Fig. 19, each electrode wire 10 is connected to a pad 80, and the pad 80, in turn, is connected to the external circuit 90; that is, the electrode wire 10 is connected to the external circuit 90 through the pad 80 connected thereto. It should be noted that the sizes of various parts shown in Fig. 19 are only exemplary, and should not be regarded as limitations on the embodiment of the present disclosure. For example, in order to facilitate the connection with the external circuit 90, all the linear structures LS of all the flexible neural electrodes included in the flexible neural electrode array are located at the same side of an array formed by the composite spiral structures CSS. However, the embodiment of the present disclosure is not limited thereto, and all the linear structures LS of all the flexible neural electrodes included in the flexible neural electrode array can also be located at two opposite sides, two adjacent sides, three sides or multiple sides of an array formed by the composite spiral structures CSS, which can be flexibly designed according to the actual situation.

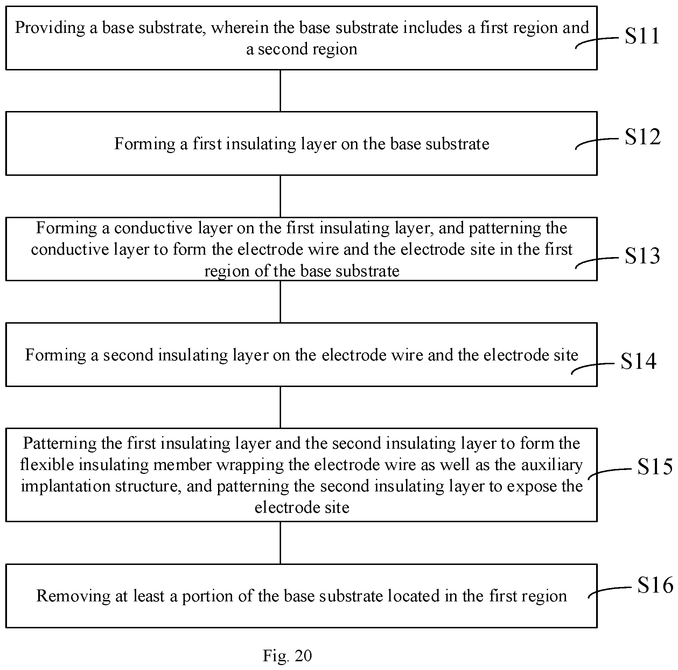

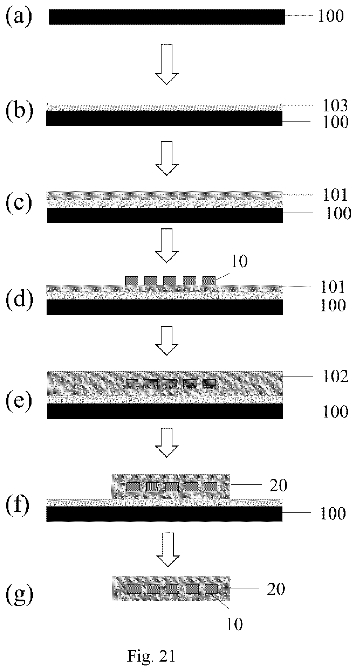

According to the embodiment of the present disclosure, a manufacturing method is also provided, which can be used for manufacturing the implantable stretchable flexible neural electrode, the implantable stretchable flexible neural electrode group and the implantable stretchable flexible neural electrode array as described above. As an example, the manufacturing method of the implantable stretchable flexible neural electrode will be described below; it should be noted that this method can be used to manufacture the implantable stretchable flexible neural electrode group and the implantable stretchable flexible neural electrode array. Fig. 20 is a flowchart of a manufacturing method of an implantable stretchable flexible neural electrode according to an embodiment of the present disclosure; Fig. 21 is a schematic cross-sectional view of various steps in the manufacturing method of the implantable stretchable flexible neural electrode according to the embodiment of the present disclosure. According to an embodiment of the present disclosure, the manufacturing method of the implantable stretchable flexible neural electrode includes the following steps: step S11, providing a base substrate 100, wherein the base substrate 100 includes a first region and a second region, as shown in the cross-sectional view (a) of Fig. 21; S12, forming a first insulating layer 101 on the base substrate 100, as shown in the cross-sectional view (c) of Fig. 21; S13, forming a conductive layer on the first insulating layer 101, and patterning the conductive layer to form the electrode wire 10 and the electrode site 30 in the first region of the base substrate 101, as shown in the cross-sectional view (d) of Fig. 21; step S14, forming a second insulating layer 102 on the electrode wire 10 and the electrode site 30, as shown in the cross-sectional view (e) of Fig. 21; step S15, patterning the first insulating layer 101 and the second insulating layer 102 to form the flexible insulating part 20 wrapping the electrode wire 10, the auxiliary implantation structure 40, as well as the spiral structure SS of the electrode wire 10 and the flexible insulating part 20, and patterning the second insulating layer 102 to expose the electrode site 30, as shown in the cross-sectional view (f) of Fig. 21; and step S16, removing at least a portion of the base substrate 100 located in the first region, as shown in the cross-sectional view (g) of Fig. 21. For example, in step S11, the base substrate 100 is a silicon wafer or a glass sheet; providing the base substrate 100 includes pretreating the base substrate 100, for example, the base substrate 100 is cleaned with acetone and isopropanol, then is cleaned with water and oven-dried, and then is cleaned with oxygen plasma. For example, in step S16, the portion of the base substrate 100 located in the second region is retained or removed; further preferably, the portion of the base substrate 100 located in the second region is retained so as to reduce the operational difficulty of removing the base substrate 100 and ensure the structural stability of the pad 80 in the second region. For example, the "patterning" process in steps S13 and S15 includes coating, exposure and development of photoresist to form a photoresist pattern, and forming a desired pattern for each layer by using the photoresist pattern as a mask.

For example, according to the embodiment of the present disclosure, the manufacturing method of the implantable stretchable flexible neural electrode further includes: before forming the first insulating layer 101 on the base substrate 100, forming a sacrificial layer 103 at least in the first region of the base substrate 100, as shown in the cross-sectional view (b) of Fig. 21; removing at least a portion of the base substrate 100 located in the first region includes: removing the sacrificial layer 103 and cutting the base substrate 100 to remove at least a portion of the base substrate 100 located in the first region. For example, the thickness of the sacrificial layer 103 is 10 nm to 1000 nm, for example, 10 nm, 30 nm, 50 nm, 70 nm, 100 nm, 300 nm, 500 nm, 700 nm or 1000 nm; further preferably, 100 nm. For example, the material of the sacrificial layer 103 includes at least one of aluminum, PMMA and nickel; further preferably, aluminum.

For example, according to the embodiment of the present disclosure, step S13 further includes: patterning the conductive layer to form the pad 80 in the second region of the base substrate 100 while forming the electrode wire 10 and the electrode site 30 in the first region of the base substrate 100, wherein the electrode wire 10 is electrically connected to the pad 80; and step S15 further includes: patterning the second insulating layer 102 to expose the pad 80. For example, the size of the pad 80 is (0.1 to 4) mm× (0.1 to 4) mm, for example, 0.1 mm×0.1 mm, 0.2 mm×0.1 mm, 1 mm×2 mm, 3.5 mm×3.5 mm, 3.7 mm×4 mm, 4 mm×4 mm or 4 mm×3.7 mm, etc.; further preferably, 0.1 mm × 0.2 mm.

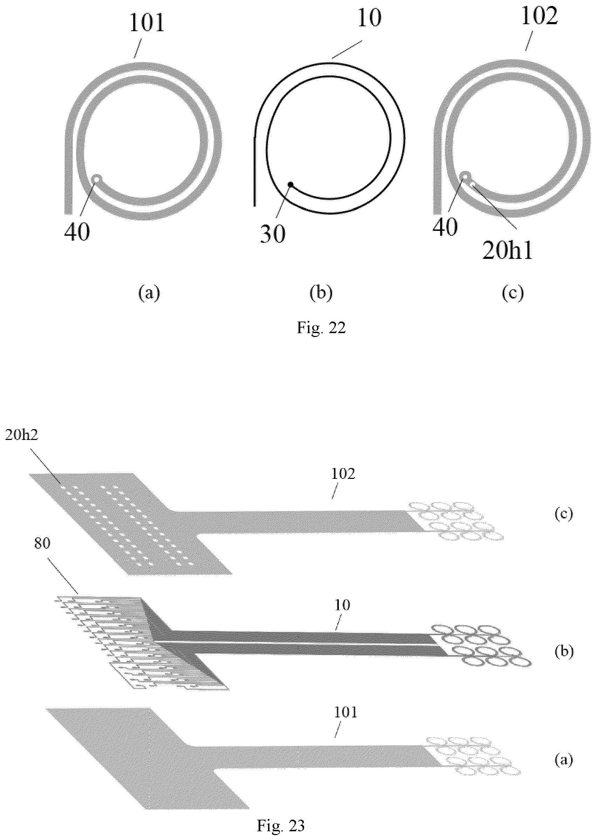

Fig. 22 is an exploded view of an implantable stretchable flexible neural electrode which is manufactured according to an embodiment of the present disclosure. Fig. 22 (a) shows the patterned first insulating layer 101, Fig. 22 (b) shows the electrode wire 10 and the electrode site 30 connected thereto, and Fig. 22 (c) shows the patterned second insulating layer 102. Referring to Fig. 22, the patterned second insulating layer 102 includes an opening 20h1 to expose the electrode site 30.

Fig. 23 is an exploded view of an implantable stretchable flexible neural electrode array which is manufactured according to an embodiment of the present disclosure. Fig. 22 (a) shows the patterned first insulating layer 101, Fig. 22 (b) shows the electrode wire 10 and the pad 80 connected thereto, and Fig. 22 (c) shows the patterned second insulating layer 102. Referring to Fig. 22, the patterned second insulating layer 102 includes an opening 20h2 to expose the pad 80. Fig. 22 is the exploded view of each of a plurality of flexible neural electrodes included in the flexible neural electrode array of Fig. 23.

The technical effects of the implantable stretchable flexible neural electrode, the implantable stretchable flexible neural electrode group and the implantable stretchable flexible neural electrode array manufactured according to the method of the embodiment of the present disclosure can refer to the above description, and will not be repeated here.

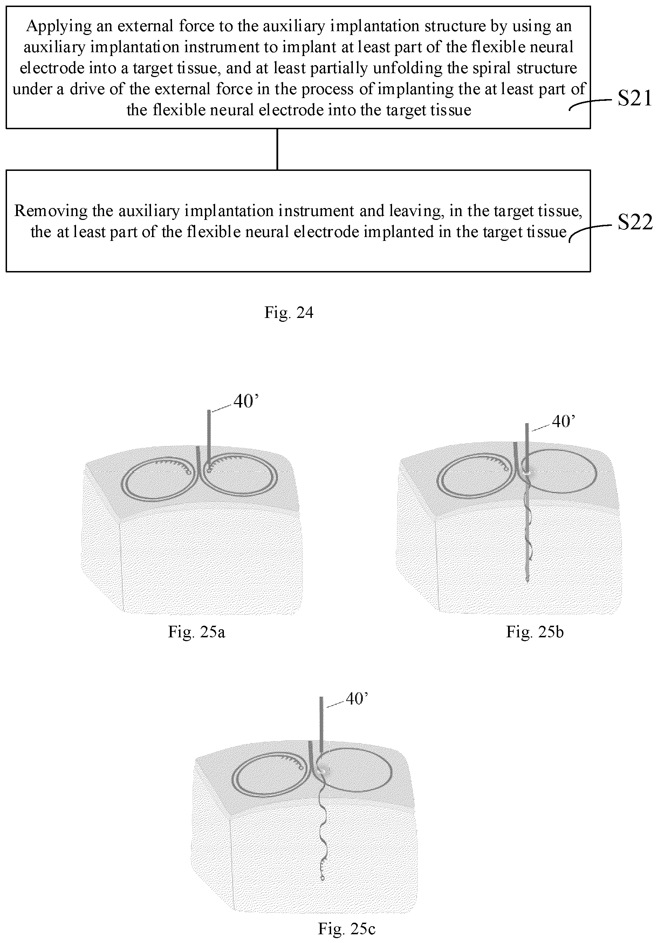

According to the embodiment of the present disclosure, an implantation method is also provided, which can be used for implanting the implantable stretchable flexible neural electrode, the implantable stretchable flexible neural electrode group, and the implantable stretchable flexible neural electrode array as described above into the target tissue. As an example, the implantation method of the implantable stretchable flexible neural electrode will be described below; it should be noted that this method can be used to implant the implantable stretchable flexible neural electrode group and the implantable stretchable flexible neural electrode array into the target tissue. Fig. 24 is a flowchart of an implantation method of an implantable stretchable flexible neural electrode according to an embodiment of the present disclosure; Figs. 25a-25c are perspective views of various steps in the implantation method of the implantable stretchable flexible neural electrode according to the embodiment of the present disclosure. According to the embodiment of the present disclosure, the implantation method of the implantable stretchable flexible neural electrode includes the following steps: step S21, applying an external force to the auxiliary implantation structure 40 by using an auxiliary implantation instrument 40' to implant at least part of the flexible neural electrode into a target tissue, and at least partially stretching the spiral structure SS under a drive of the external force in the process of implanting the at least part of the flexible neural electrode into the target tissue, as shown in Fig. 25b; and step S22, removing the auxiliary implantation instrument 40' and leaving, in the target tissue, the at least part of the flexible neural electrode implanted in the target tissue, as shown in Fig. 25c. For example, according to an embodiment of the present disclosure, before step S21, the implantation method further includes: assembling the auxiliary implantation instrument 40' with the auxiliary implantation structure 40, as shown in Fig. 25a. As an example, in Figs. 25a to 25c, the auxiliary implantation structure 40 is a through hole, and the auxiliary implantation instrument 40' cooperating with the auxiliary implantation structure 40 has a protrusion engaged with the through hole. However, the embodiment of the present disclosure is not limited thereto, and in the case where the auxiliary implantation structure 40 is a protrusion, the auxiliary implantation instrument 40' cooperating with the auxiliary implantation structure 40 may have a hole or a groove engaged with the protrusion.

According to the implantation method of the embodiment of the present disclosure, all the electrode wires 10 can be stretched and implanted into the target tissue in situ, thereby improving the implantation density and reducing the difficulty of the implantation process.

What have been described above are only exemplary embodiments of the present disclosure, and are not used to limit the scope of protection of the present disclosure, which is determined by the appended claims.

## Claims

1. An implantable stretchable flexible neural electrode, comprising an electrode wire and a flexible insulating part wrapping the electrode wire, wherein
the flexible neural electrode comprises a spiral structure of the electrode wire and the flexible insulating part, wherein the spiral structure is stretchable; an electrode site and an auxiliary implantation structure are provided at the spiral structure; the electrode site is electrically connected to the electrode wire and exposed from the flexible insulating part; and the auxiliary implantation structure is configured to drive the spiral structure to stretch under an action of external force.

2. The implantable stretchable flexible neural electrode according to claim 1, wherein the spiral structure is configured to be stretchable in any direction.

3. The implantable stretchable flexible neural electrode according to claim 1 or 2, wherein
the spiral structure comprises a plurality of circles, wherein any two adjacent circles among the plurality of circles are an inner circle and an outer circle, respectively, the outer circle surrounds the inner circle, and a termination end of the outer circle is connected to an initial end of the inner circle.

4. The implantable stretchable flexible neural electrode according to claim 3, wherein
the auxiliary implantation structure is provided at an innermost circle among the plurality of circles.

5. The implantable stretchable flexible neural electrode according to claim 4, wherein
the flexible insulating part is provided with a protruding part extending beyond the electrode wire, and the auxiliary implantation structure is a through hole, a groove or a protrusion provided at the protruding part.

6. The implantable stretchable flexible neural electrode according to claim 5, wherein a position mark is provided on the protruding part.

7. The implantable stretchable flexible neural electrode according to any one of claims 3-6,
wherein each of the plurality of circles comprises a plurality of curved portions connected in sequence.

8. The implantable stretchable flexible neural electrode according to any one of claims 1-7,
further comprising a linear structure of the electrode wire and the flexible insulating part,
wherein the linear structure is connected to the spiral structure.

9. The implantable stretchable flexible neural electrode according to claim 8, wherein
the linear structure comprises an auxiliary electrode wire wrapped by the flexible insulating part; and
in the linear structure, the auxiliary electrode wire is electrically connected to the electrode wire.

10. The implantable stretchable flexible neural electrode according to any one of claims 1-9, further comprising an anchoring structure, wherein
an angle is provided between the anchoring structure and the electrode wire, and the anchoring structure is configured to interact with a target tissue after the flexible neural electrode is implanted into the target tissue so as to prevent the flexible neural electrode from moving relative to the target tissue.

11. The implantable stretchable flexible neural electrode according to claim 10, wherein an edge of the anchoring structure extending from the electrode wire in a direction away from the electrode wire is in an arc shape, and the arc shape protrudes towards the auxiliary implantation structure.

12. The implantable stretchable flexible neural electrode according to claim 10 or 11,
wherein the anchoring structure comprises an anchoring electrode wire covered by the flexible insulating part, and the electrode site is electrically connected to the electrode wire through the anchoring electrode wire.

13. The implantable stretchable flexible neural electrode according to any one of claims 1-12, wherein
a material of the electrode wire and a material of the electrode site comprise at least one of gold, platinum and iridium; and
a material of the flexible insulating part comprises at least one of polyimide, parylene and SU-8 photoresist.

14. The implantable stretchable flexible neural electrode according to any one of claims 1-13, further comprising an adhesive layer provided between the electrode wire and the flexible insulating part.

15. The implantable stretchable flexible neural electrode according to any one of claims 1-14, comprising:
a plurality of electrode wires wrapped by the flexible insulating part and insulated from each other; and
a plurality of electrode sites exposed from the flexible insulating part, electrically connected to the plurality of the electrode wires in one-to-one correspondence, and spatially separated from each other.

16. The implantable stretchable flexible neural electrode according to claim 15, wherein
the plurality of electrode wires are located at a same layer; or
the plurality of electrode wires are located at a plurality of layers arranged in a stacked manner, and each of the plurality of layers comprises at least one of the plurality of electrode wires.

17. The implantable stretchable flexible neural electrode according to claim 16, wherein
any two adjacent layers among the plurality of layers are a first layer and a second layer;
in a stacking direction of the plurality of layers, the electrode wire located at the first layer at least partially overlaps or at least partially does not overlap with the electrode wire located at the second layer.

18. The implantable stretchable flexible neural electrode according to any one of claims 15-17, wherein the plurality of electrode sites are located at a same side or at different sides of the flexible neural electrode.

19. An implantable stretchable flexible neural electrode group, comprising a plurality of implantable stretchable flexible neural electrodes according to any one of claims 1-18, wherein
the plurality of flexible neural electrodes are insulated from each other;
a plurality of spiral structures of the plurality of flexible neural electrodes forms a composite spiral structure, and in a same circle of the composite spiral structure, parts of the plurality of spiral structures located in the same circle of the composite spiral structure are sequentially arranged from inside to outside.

20. The implantable stretchable flexible neural electrode group according to claim 19,
wherein a plurality of electrode sites of the plurality of flexible neural electrodes are spatially separated from each other.

21. The implantable stretchable flexible neural electrode group according to claim 19 or 20, wherein
the composite spiral structure comprises a plurality of circles, wherein any two adjacent circles among the plurality of circles are an inner circle and an outer circle, respectively, the outer circle surrounds the inner circle, and a termination end of the outer circle is connected to an initial end of the inner circle;
in each of the plurality of circles of the composite spiral structure, corresponding parts of the plurality of spiral structures are sequentially arranged from inside to outside in a same order.

22. The implantable stretchable flexible neural electrode group according to any one of claims 19-21, wherein
a plurality of auxiliary implantation structures of the plurality of flexible neural electrodes are integrated into a common auxiliary structure, and the common auxiliary structure is configured to drive a plurality of spiral structures of the plurality of flexible neural electrodes to stretch synchronously under an action of external force.

23. The implantable stretchable flexible neural electrode group according to claim 22,
wherein a plurality of flexible insulating parts of the plurality of flexible neural electrodes are at least partially connected to each other.

24. The implantable stretchable flexible neural electrode group according to any one of claims 19-21, wherein a plurality of auxiliary implantation structures of the plurality of flexible neural electrodes are spatially separated from each other.

25. An implantable stretchable flexible neural electrode array, comprising a plurality of flexible neural electrodes according to any one of claims 1-18, wherein
the plurality of flexible neural electrodes are insulated from each other, and a plurality of spiral structures of the plurality of flexible neural electrodes are arranged in an array.

26. The implantable stretchable flexible neural electrode array according to claim 25, wherein
each of the plurality of flexible neural electrodes comprises a linear structure of the electrode wire and the flexible insulating part, and the linear structure is connected to the spiral structure.

27. An implantable stretchable flexible neural electrode array, comprising a plurality of flexible neural electrode groups according to any one of claims 19-24, wherein
a plurality of composite spiral structures of the plurality of flexible neural electrode groups are arranged in an array.

28. The implantable stretchable flexible neural electrode array according to claim 27, wherein
each of all the flexible neural electrodes included in the plurality of flexible neural electrode groups comprises a linear structure of the electrode wire and the flexible insulating part, and the linear structure is connected to the spiral structure.

29. A manufacturing method of the implantable stretchable flexible neural electrode according to any one of claims 1-18, or the implantable stretchable flexible neural electrode group according to any one of claims 19-24, or the implantable stretchable flexible neural electrode array according to any one of claims 25-28, comprising:
providing a base substrate, wherein the base substrate comprises a first region and a second region;
forming a first insulating layer on the base substrate;
forming a conductive layer on the first insulating layer and patterning the conductive layer to form the electrode wire and the electrode site in the first region of the base substrate;
forming a second insulating layer on the electrode wire and the electrode site;
patterning the first insulating layer and the second insulating layer to form the auxiliary implantation structure and the flexible insulating part wrapping the electrode wire, and patterning the second insulating layer to expose the electrode site; and
removing at least a portion of the base substrate located in the first region.

30. The manufacturing method according to claim 29, further comprising:
forming a sacrificial layer at least in the first region of the base substrate before forming the first insulating layer on the base substrate; and
the removing at least a portion of the base substrate located in the first region comprises:
removing the sacrificial layer and cutting the base substrate to remove at least a portion of the base substrate located in the first region.

31. The manufacturing method according to claim 29 or 30, wherein
patterning the conductive layer to form a pad in the second region of the base substrate while forming the electrode wire and the electrode site in the first region of the base substrate, wherein the electrode wire is electrically connected to the pad; and
the manufacturing method further comprises: patterning the second insulating layer to expose the pad.

32. An implantation method of the implantable stretchable flexible neural electrode according to any one of claims 1-18 or the implantable stretchable flexible neural electrode group according to any one of claims 19-24 or the implantable stretchable flexible neural electrode array according to any one of claims 25-28, comprising:
applying an external force to the auxiliary implantation structure by using an auxiliary implantation instrument to implant at least part of the flexible neural electrode into a target tissue, and at least partially stretching the spiral structure under a drive of the external force during a process of implanting the at least part of the flexible neural electrode into the target tissue; and
removing the auxiliary implantation instrument and leaving, in the target issue, the at least part of the flexible neural electrode implanted in the target tissue.
